# EUROPEAN PATENT SPECIFICATION

(11) **EP 3 534 833 B1**
(45) Date of publication and mention of the grant of the patent: **28.06.2023**
(21) Application number: 17913336.8
(22) Date of filing: 06.11.2017
(51) Int. Cl.: A61F 2/00, B82Y 40/00, A61L 27/26, A61L 27/54, A61L 27/56, A61L 27/58

(54) **AN ARTIFICIAL BIOMIMETIC NERVOUS TISSUE SCAFFOLD AND PRODUCTION METHOD THEREOF**
KÜNSTLICHES BIOMIMETISCHES NERVENGEWEBEGERÜST UND HERSTELLUNGSVERFAHREN DAFÜR
ÉCHAFAUDAGE DE TISSU NERVEUX BIOMIMÉTIQUE ARTIFICIEL ET SON PROCÉDÉ DE PRODUCTION

(30) Priority: 07.11.2016 TR 201615825
(43) Date of publication of application: 11.09.2019
(73) Proprietor: T.C Medipol Üniversitesi, Kavacik/Istanbul (TR)
(72) Inventor: ALTUN, Esra, 34240 Gaziosmanpasa/Istanbul (TR); GÜNDÜZ, Oguzhan, Tuzla/Istanbul (TR); EKREN, Nazmi, Bakirköy/Istanbul (TR); SENGIL, Ahmet Zeki, Üsküdar/Istanbul (TR)
(74) Representative: Simsek, Meliha Merve
(86) International application number: PCT/TR2017/000122
(87) International publication number: WO 2018/231161

(56) References cited:
- WO-A2-2006/042287
- WO-A2-2008/100534
- WO-A2-2009/094225
- US-A1- 2006 002 978
- US-A1- 2007 010 831
- US-A1- 2011 086 236
- US-A1- 2014 363 890
- DE OLIVEIRA BARUD HÉLIDA GOMES ET AL: "A multipurpose natural and renewable polymer in medical applications: Bacterial cellulose", CARBOHYDRATE POLYMERS, APPLIED SCIENCE PUBLISHERS, LTD. BARKING, GB, vol. 153, 19 July 2016 (2016-07-19), pages 406-420, XP029703692, ISSN: 0144-8617, DOI: 10.1016/J.CARBPOL.2016.07.059

## Description

### Technical Field

The present invention relates to a biodegradable and biocompatible three-dimensional artificial biomimetic nerve tissue scaffold and method of manufacturing the same, which enables the tissues to regenerate and form healthy tissue and support damaged tissue.

### Prior Art

Recent developments in tissue engineering applications aim to create healthy tissues by supporting damaged body tissues using artificial tissue scaffolds obtained from synthetic or biopolymers .Nano- sized fibers are obtained by electrospinning method that is one of the production techniques developed for this purpose and artificial tissue scaffolds are formed by accumulating these fibers on top of each other.

One of the systems that focuses on tissue engineering applications today is the human nervous system. Numerous studies have been conducted to support nerve cell loss resulting from nervous system damage with new growing cells.However, the polymers and production methods used in these studies are costly and require a long process.

In a study conducted by Mobarakeh et al in 2008 .(L. Ghasemi-Mobarakeh, M. P. Prabhakaran, M. Morshed, M. H. Nasr- Esfahani, S.Ramakrishna Biomaterials 2008, 29, 9) , the nanofiber structure obtained by electrospinning technique using polycaprolactone and gelatin polymers was used as a tissue scaffold and cell growth was observed.

In the study conducted by Jha et al. In 2010 (BSJha, RJ Colello, J. R. Bowman, SA Sell, KD Lee, J.W. Bigbee, GL Bowlin, WN Chow, BE Mathern, DG Simpson, Acta Biomaterialia 2011, 7, 203-215) the product obtained by air gap electrospinning of polycaprolactme polymer has also been observed to be slightly effective on restructuring of the nerve in tissue wear However, air gap electrospinning method is more costly and difficult than simple electrospinning.

In a study by Yu et al conducted in 2011, (W.Yu, W. Zhao, C. Zhu, BMC Neurosci. 2011, 12, 1471), collagen and polycaprolactone polymers were used in the electrospinning process and nano structured fibers were obtained.The tissue scaffold obtained in the study gave an effective result in the growth of Schwann cells in the peripheral nervous system.However, collagen used in tissue scaffold production is a very expensive material.

In a study by Zhu et al done in 2014, (W.Zhu, C. O'Brien, J. B. O'Brien, L. G. Zhang, Nanomedicine 2014, 9, 75), artificial nerve tissue scaffolds were produced by using electrospinning technique and three-dimensional printer methods, and biochemical markers were added to investigate nerve tissue regeneration.However, the method used in this study increases the production cost of the tissue scaffold.

International patent document WO2006096791, which is known in the art, discloses a tissue scaffold having a plurality of uniaxially oriented nanofiber structures made of at least one synthetic polymer that provides tissue renewal. In a preferred embodiment of the invention described in said document, the tissue scaffold may be placed in a patient who needs a nerve or other tissue regeneration. In a preferred embodiment of the invention described in said document, the tissue scaffold consists of layers and the electrospinning process to obtain two or more uniaxially oriented nanofibers is achieved by following the steps of stacking the nanofiber films to form a 3D tissue scaffold.In order to obtain a tissue scaffold in the said invention, biodegradable or non-synthetic polymers or mixtures thereof may be used. In one embodiment of the invention described in said document, the tissue scaffold comprises at least one biochemical agent. In one embodiment, it is mentioned that the biochemical agent is growth factor or differentiation factor.It has also been mentioned that the tissue scaffold may contain liquid microtubules or nanoparticles for controlled release of the biochemical agent.

The international patent numbered WO2008093341 refers to a tissue scaffold comprising a large number of particles with a porous structure containing electrospun polynanofibers. The document also describes tissue scaffold production methods, tissue production methods, and methods of using tissue scaffolds for tissue restructuring.Electrospinning technique is used to obtain the tissue scaffold in the subject, and the polynanofibers obtained by the said technique are composed of natural biodegradable polymers and/or synthetic biodegradable polymers. In the context of said disclosure, the natural biodegradable polymers for tissue scaffold production include silk and collagen as natural biodegradable polymers and; polycaprolactone as synthetic biodegradable polymers and also biochemical materials such as bacterial cellulose and growth factor can also be used.

US 2014/363890 A1 discloses nerve regeneration devices comprising fibrils obtained by electrospinning a solution of PCL comprising beads of bacterial cellulose.

### Brief Description of the Invention

The object of this invention is to realize a biodegradable and biocompatible three-dimensional artificial biomimetic nerve tissue scaffold for the self-renewal of the damaged and non-renewable central nervous system and a production method thereof. Another object of the invention is to realize an artificial biomimetic nerve tissue scaffold and a production method thereof which is smoothly accepted by the nervous system and which allows the mesenchimal nerve cells to regenerate by producing new nerve cells and which is destroyed by the body after new cells are produced and does not harm living tissue.

The objectives of the present invention are achieved by the product as defined in claim 1 and by the method as defined in claim 8.

### Detailed Description of the Invention

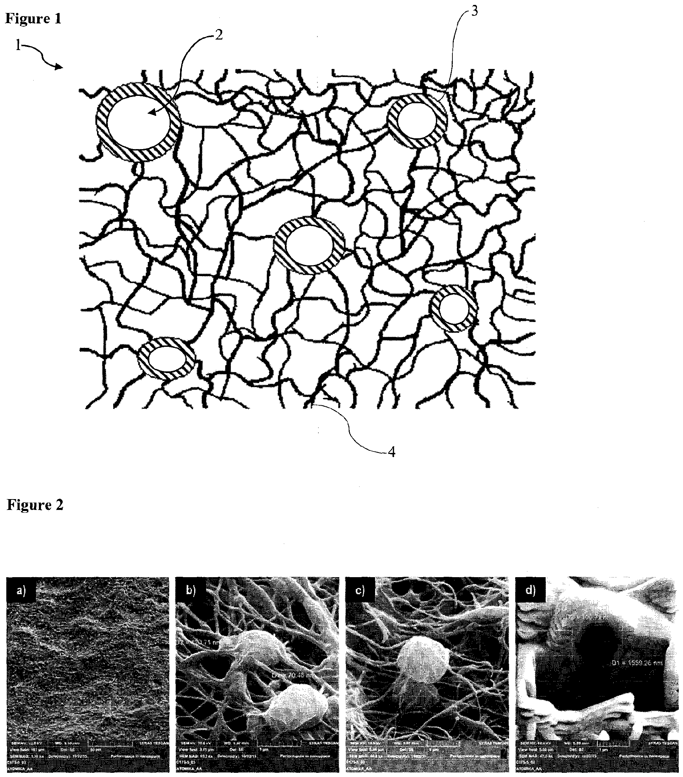

"Artificial Biomimetic Nerve TissueScaffold and Manufacturing Method (100)"; performed to achieve the purpose of this invention is illustrated in the attached figures, wherein
**Figure 1****.** The schematic representation of the tissue scaffold formed by the nanofiber structure and the hollow capsule structure obtained by the method of the invention
**Figure-2.** Scanning electron microscopy (SEM) images of nerve tissue scaffold that is the subject of the invention
   (a) image of the tissue scaffold according to the invention at 100X magnification;
   (b) At 1.00KX magnification of the nanofiber structure and the capsule within the nanofiber structure;
   (c) At 1.00 KX magnificationone capsule structure and the appearance of the nanofiber branches in the vicinity;
   (d) At 2.00 KX magnification, the image of the nanofiber and rhe hollow internal structure of the capsule in the structure.
**Figure-3.** As a result of the in vitro cell culture assay of the invention the resulting cell viability result plots of the resulting PCL and BCs / PCL tissue scaffolds.
   (*** P value & 1t; 0.05):
   (a) 24 hours;
   (b) 48 hours;
   (c) 72 hours.
**Figure-4.** A flow diagram of a tissue scaffold production method according to present invention.

The parts in the figures are numbered individually and their correspondences are given below.
1. Tissue scaffold
2. Cavity
3. Capsule
4. Nanofiber structure

A three-dimensional artificial biomimetic nerve tissue scaffold (1), that is biodegradable and biocompatible, which allows self-renewal of damaged tissues in essence comprises a nanofiber structure comprising nanofibers obtained by electrospinning technique using bacterial cellulose biopolymers (BCs) and polycaprolactone (PCL) synthetic polymer and in between nanofibers at least one nano / microcapsule (3) containing at least one cavity (2).

In the tissue scaffold of the invention (1), the nano / microcapsules (3) located between the nanofibers have a diameter of 100 nm to 1.6 µm. The diameter of the cavity (2) in said nano / microcapsules (3) is 80 nm to 1.5 µm in diameter. In the tissue scaffold (1) of the present invention, the nanofibers in the vicinity of the nano/microcapsule (3) in the nanofiber structure (4) have a diameter of 70 nm to 121 nm

In a preferred embodiment of the invention, at least one biochemical marker is added to the cavity (2) located in at least one of the nano/microcapsules (3) between the nanofibers constituting the inventive tissue scaffold (1). The biochemical markers added to the cavity (2) in the inventive tissue scaffold (1) provide convenience in the examination of tissue damage by biomedical devices and microscopes. As a biochemical marker; Myelin Basic Protein (MBP), Neuron Specific Enolase (NSE), Tau Protein, 14-3-3 Protein, S-100 Protein and Glial Fibrillary Acidic Protein can be used.

In another preferred embodiment of the invention, at least one growth factor is added to the cavity (2) contained in at least one of the nano / microcapsules (3) present between the nanofibers constituting the tissue scaffold of the invention (1). In the tissue scaffold of the invention, the growth factors (neurotrophines) added to the cavity (2) present in said tissue scaffold are dimeric polypeptide structured growth factor family that enables viability, growth, reproduction and function on neurons, stabilized synapses, controlling synaptic function and synaptic plasticity regulates axon and dendritic branching, and is divided into six subclasses, namely; nerve growth factor (NGF), Brain Derived Neurotrophilic Factor (BDNF), Neurotrophin-3(NT-3), Neurotrophin-4/5 (NT-4/5), Neurotrophin-6 (NT-6) and Neurotrophin-7 (NT-7).

The nervous system smoothly accepts the artificial tissue scaffold (1) of the invention because the artificial tissue scaffold (1) of the present invention is a nanofiber structure (4) containing biopolymer bacterial cellulose and biocompatible and biocompatible synthetic polymer polycaprolactone.

The artificial tissue scaffold (1) of the invention comprising the nanofiber structure (4) obtained by combining the bacterial cellulose biopolymer and the polycaprolactone synthetic polymer using the electrospinning method comprises capsules (3) containing the cavity (2) in the nano and/or micro dimension .In the artificial tissue scaffold (1) of the invention biochemical markers and/or growth factors are added to the cavity (2) inside at least one of the capsules (3) that are bonded to each other via nano and micro-sized nanofibers. This way, transport and controlled release of biochemical markers and/or growth factors added to nano and micro sized capsules (3) are achieved.

Scanning electron microscope (SEM) images of the nerve tissue scaffold (1), nano/micro capsules (3) and nanofiber structure (4) are shown in FIG.2Figure 2d shows a SEM image of a capsule (3) located on the nerve tissue scaffold (1) of the invention, the surface of which is crossed by the FEG-SEM device.

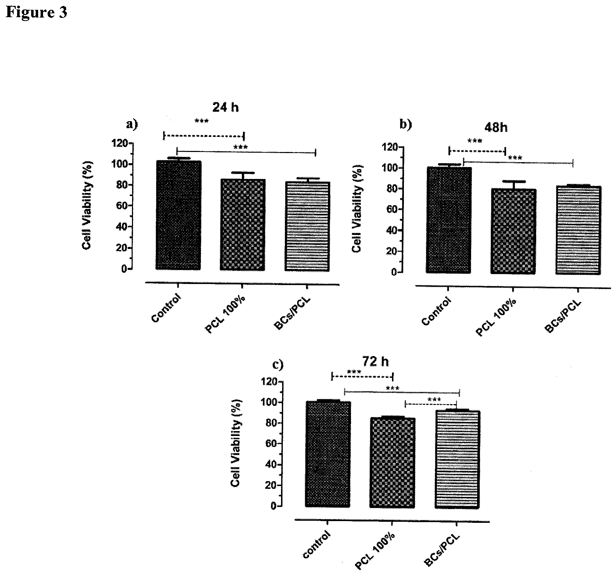

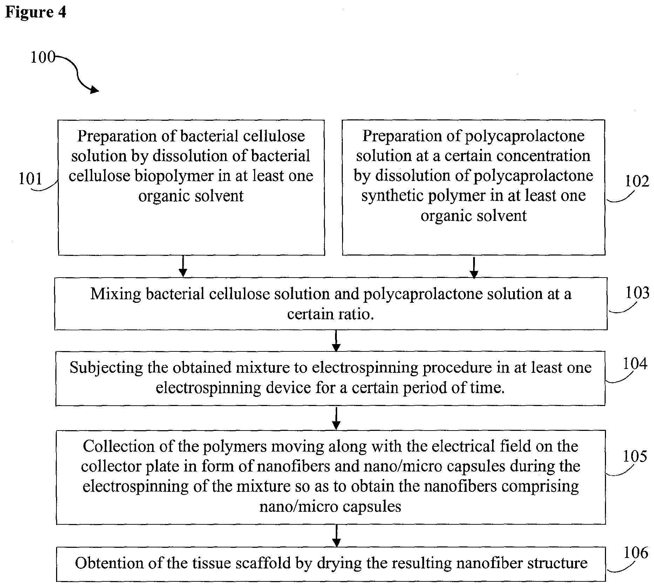

In FIG 3 a graph showing the results of of the proliferation and cell viability test of tissue scaffolds (BCs / PCL) (1) and pure polycaprolactone (PCL) L929 on mouse fibroblast cells in given. According to the in-vitro cell culture test result, the cell potency in the inventive tissue scaffold (1) is 84% after 24 hours, 83% after 48 hours and 93% after 72 hours.

The invention relates to an artificial biomimetic nerve tissue scaffold production method (100) which comprises the steps of;
- preparing a bacterial cellulose solution at a given concentration by dissolving the bacterial cellulose biopolymer in at least one organic solvent (101) ,
- preparing a polycaprolactone solution at a certain concentration by dissolving the polycaprolactone synthetic polymer in at least one organic solvent (102),
- mixing the bacterial cellulose solution and the polycaprolactone solution at a specific ratio (103),
- Subjecting the mixture containing the bacterial cellulose solution and the polycaprolactone solution for a certain period of time to electrospinning process in at least one electrospinning device having at least one needle and one collector plate by adjusting variables such as the voltage, distance between the collector plate and needle and flow rate that has impact on nanofiber diameter and morphology (104),
- Collection of the polymers moving along with the electrical field on the collector plate in form of nanofibers and nano/micro capsules during the electrospinning of the mixture so as to obtain the nanofibers (4) comprising nano/micro capsules (3)
- Obtention of the tissue scaffold (1) by drying the resulting nanofiber structure (4) (106).

In a preferred embodiment of the tissue scaffold production method (100) of the present invention, the bacterial cellulose biopolymer is dissolved in the organic solvent so the bacterial cellulose is present in an amount of 5% by weight in the solvent. For this, the bacterial cellulose biopolymer is cut into small pieces, and placed in a solvent that is preferably containing dimethylformamide (DMF) and tetrahydrofuran (THF) 50:50 by weight, and subjected to ultrasonic water bath for 15 days to prepare a bacterial cellulose solution (101).

In a preferred embodiment of the method of the present invention, the polycaprolactone polymer is added to a solvent so as to have 5% by weight, wherein such solvent is preferably the chloroform and dimethylformamide (DMF) solvent, preferably in a ratio of 50:50 by weight .Q^(MThe polycaprolactone solution is then prepared by stirring in a magnetic stirrer, preferably for 1.5 hours (102).

In the method of the invention, the preparation of the bacterial cellulose solution (100) and the preparation of the polycaprolactone solution (102) are followed by a mixing of both solutions preferably in a ratio of 50:50 by weight (103).

In the tissue scaffold production method of the invention (100), the mixture obtained by the mixing (103) of the bacterial cellulose solution and the polycaprolactone solution is transferred into the syringe and transferred to the pump section which then progresses the predetermined amount of polymer in the electrospinning machine for a predetermined time The flow rate of the mixture loaded in the electrospinning machine preferably has a value of 1 ml/h. Subsequently , the voltage of the electricity applied to provide nanofiber production containing the nano / microcapsule (3) is preferably about 28 kV and the distance between the needle and the collector plate is about 13 cm.In the inventive tissue scaffold production method of the invention (100), after preferably 5 hours of electrospinning procedure; nanofibers containing nano/microcapsules (3) in between are collected on the collector plate and the nanofiber structure (4) containing the nano / microcapsules (3) is obtained.

In the tissue scaffold production method (100) of the present invention, the nanofiber structure (4) comprising the nano/micro capsule (3) is dried preferably in the oven for 1 day so as to obtain a tissue scaffold (1) (106).

The bacterial cellulose biopolymer used in the nerve tissue scaffold of the invention (1) is readily accepted by the tissues and can be destroyed by body cells after a certain period of time. The bacterial cellulosic structure used in the nerve tissue scaffold of the invention (1) is structurally similar to the collagen used in the production of tissue scaffold known in the state of the art; the however its effect on immunological reactivity is better than that of collagen.

The bacterial cellulose used in the tissue scaffold of the invention (1) can be produced in a short time at low cost .Thus , the use of bacterial cellulose instead of the collagen, which is quite expensive, reduces the production cost of the nerve tissue scaffold (1). Furthermore, the bacterial cellulose used in the tissue scaffold of the invention (1) facilitates the acceptance of the artificial tissue scaffold produced by the method of the present invention (1) by the damaged nerve tissue, and greatly enhances cell growth and neural regeneration.

Polycaprolactone used in the tissue scaffold of the present invention (1) is a biodegradable and biocompatible polymer produced from renewable sources. Thus, the tissue scaffold (1) obtained by the invention is easily accepted by the tissues and can be easily destroyed by the body cells after a certain period of time.

The nanofiber structure (4) is obtained in a single step by means of electrospinning technique using bacterial cellulose and polycaprolactone for the production of the nerve tissue scaffold of the invention (1) This reduces the production cost of nerve tissue scaffold (1).

The nano/microcapsule (3) structures within the nanofiberstructure (4) of the nerve tissue scaffold (1) of the invention facilitate transport of growth factor and/or biochemical markers to the damaged nerve tissue .Additionally , the said nano/microcapsules (3) having the cavity (2) are able to release more due to the fact that they have more growth factor and biochemical marker storage capacity.This positively affects cell growth.

Around these basic concepts, it is possible to develop a wide variety of applications for the inventive subject "An Artificial Biomimetic Neural Tissue Scaffold and Production Method thereof (100)" and the invention is not limited to the examples disclosed herein, but is essentially as specified in the claims.

## Claims

1. A three dimensional artificial neural tissue scaffold (1) which is biodegradable and biocompatible, enabling self-renewal of damaged tissues comprising a nanofiber structure (4) and further comprising at least one nano / microcapsule (3) containing at least one cavity (2), wherein the three dimensional artificial neural tissue scaffold is obtained by a method comprising the steps of:
- preparing a bacterial cellulose solution at a given concentration by dissolving bacterial cellulose biopolymer in at least one organic solvent (101),
- dissolving polycaprolactone synthetic polymer in at least one organic solvent to prepare a polycaprolactone solution at a certain concentration (102),
- mixing the bacterial cellulose solution and the polycaprolactone solution at a predetermined ratio (103),
- subjecting the mixture containing the bacterial cellulose solution and the polycaprolactone solution for a certain period of time to electrospinning process in at least one electrospinning device having at least one needle and one collector plate by adjusting variables such as the voltage, distance between the collector plate and needle and flow rate that has impact on nanofiber diameter and morphology (104),
- collection of the polymers moving along with the electrical field on the collector plate in form of nanofibers and nano/micro capsules during the electrospinning of the mixture so as to obtain the nanofibers (4) comprising nano/micro capsules (3) (105), and
- obtention of the tissue scaffold (1) by drying the resulting nanofiber structure (4).

2. A tissue scaffold (1) according to claim 1, **characterized in that** capsulees (3) are bonded to each other via nano and macrosized fibers.

3. A tissue scaffold (1) according to claim 1, **characterized in that** the nano/microcapsule (3) has a diameter of 100 nm to 1.6 µm.

4. A tissue scaffold (1) according to claim 1 or 2, **characterized in that** the cavity (2) has a diameter of 80 nm to 1.5 µm.

5. A tissue scaffold (1) according to any one of the above claims, **characterized in that** the nanofiber structure (4) comprises nanofibers having a diameter in the range of 70 nm to 121 nm.

6. A tissue scaffold (1) according to any one of the preceding claims, **characterized in that** a nano/microcapsule (3) with at least one biochemical mark attached to the cavity (2) located in at least one of the cavities (2).

7. A tissue scaffold (1) according to any one of the above claims, **characterized in that** the nano/microcapsule (3) with at least one growth factor added to the space (2) located in at least one of the cavities (2).

8. A method of producing an artificial biomimetic neural tissue scaffold **characterized in that** said method comprises the steps of - preparing a bacterial cellulose solution at a given concentration by dissolving the bacterial cellulose biopolymer in at least one organic solvent (101),
- dissolving polycaprolactone synthetic polymer in at least one organic solvent to prepare a polycaprolactone solution at a certain concentration (102)
- mixing the bacterial cellulose solution and the polycaprolactone solution at a predetermined ratio (103),
- subjecting the mixture containing the bacterial cellulose solution and the polycaprolactone solution for a certain period of time to electrospinning process in at least one electrospinning device having at least one needle and one collector plate by adjusting variables such as the voltage, distance between the collector plate and needle and flow rate that has impact on nanofiber diameter and morphology (104),
- collection (104) of the polymers moving along with the electrical field on the collector plate in form of nanofibers and nano/micro capsules during the electrospinning of the mixture so as to obtain the nanofibers (4) comprising nano/micro capsules (3) (105), and
- obtention of the tissue scaffold (1) by drying the resulting nanofiber structure (4)

9. A method of producing a nerve tissue scaffold 100 according to claim 8, **characterized in that** o bacterial cellulose solution is prepared in a solvent so as to have bacterial cellulose 5% by weight of the in the solvent (101) and polycaprolactone solution is prepared as a 5% by weight polycaprolactone polymer solution in the solvent (102).

10. A method of producing a nerve tissue scaffold (100) according to claim 8 or 9, **characterized in that** bacterial cellulose solution is prepared by adding the bacterial cellulose biopolymer to a solvent containing dimethylformamide (DMF) and tetrahydrofuran (THF) in a weight ratio of 50: 50.

11. A nerve tissue scaffold production method (100) according to any one of claims 8 to 10, **characterized in that** polycaprolactone solution is prepared by adding the polycaprolactone polymer to the solvent mixture of chloroform and dimethylformamide (DMF) mixed in a ratio of 50:50 by weight.

12. A method of producing a nerve tissue scaffold (100) according to any one of claims 8 to 11, **characterized in that** bacterial cellulose solution and the polycaprolactone solution are mixed 50:50 by weight (103).

13. A method of producing a nerve tissue scaffold (100) according to any one of claims 8 to 12, **characterized in that** the mixture containing the bacterial cellulose solution and the polycaprolactone solution is subjected to electrospinning process at a flow rate of 1 ml/hr (104).

14. A nerve tissue scaffold production method (100) according to any one of claims 8 to 13, **characterized in that** the mixture comprising the bacterial cellulose solution and the polycaprolactone solution is subjected to the electrospinning process at a voltage value of 28 kV.

15. A method of producing a nerve tissue scaffold (100) according to any one of claims 8 to 14, **characterized in that** the mixture comprising the bacterial cellulose solution and the polycaprolactone solution is subjected to electrospinning process in such a way that the distance between the needle and the collector plate is 13 cm.

16. A method of producing a nerve tissue scaffold (100) according to any one of claims 8 to 15, **characterized in that** the mixture containing the bacterial cellulose solution and the polycaprolactone solution is subjected to electrospinning process for a period of 5 hours.

## Patentansprüche

1. Dreidimensionales künstliches neurales Gewebegerüst (1), das biologisch abbaubar und biokompatibel ist, das die Selbsterneuerung von geschädigtem Gewebe ermöglicht, umfassend eine Nanofaserstruktur (4) und ferner umfassend mindestens eine Nano-/Mikrokapsel (3), die mindestens eine Kavität (2) enthält, wobei das dreidimensionale künstliche neurale Gewebegerüst durch ein Verfahren erhalten wird, das die folgenden Schritte umfasst:
- Herstellen einer Bakterienzelluloselösung in einer bestimmten Konzentration durch Auflösen des Bakterienzellulose-Biopolymers in mindestens einem organischen Lösungsmittel (101),
- Auflösen des synthetischen Polycaprolacton-Polymers in mindestens einem organischen Lösungsmittel zur Herstellung einer Polycaprolactonlösung mit einer bestimmten Konzentration (102),
- Mischen der Bakterienzelluloselösung und der Polycaprolactonlösung in einem vorgegebenen Verhältnis (103),
- Unterziehen des Gemischs, das die Bakterienzelluloselösung und die Polycaprolactonlösung enthält, für eine bestimmte Zeit dem Elektrospinnprozess in mindestens einer Elektrospinnvorrichtung mit mindestens einer Nadel und einer Kollektorplatte durch Einstellen von Variablen wie der Spannung, dem Abstand zwischen der Kollektorplatte und der Nadel und der Durchflussrate, die Auswirkungen auf den Nanofaserdurchmesser und die Morphologie haben (104),
- Sammeln der Polymere, die sich mit dem elektrischen Feld auf der Kollektorplatte in Form von Nanofasern und Nano-/Mikrokapseln während des Elektrospinnens des Gemischs bewegen, um die Nanofasern (4) zu erhalten, die Nano-/Mikrokapseln (3) umfassen (105), und
- Erhalten des Gewebegerüsts (1) durch Trocknen der entstandenen Nanofaserstruktur (4).

2. Gewebegerüst (1) nach Anspruch 1, **dadurch gekennzeichnet, dass** Kapseln (3) über nano- und makrogroße Fasern miteinander verbunden sind.

3. Gewebegerüst (1) nach Anspruch 1, **dadurch gekennzeichnet, dass** die Nano-/Mikrokapsel (3) einen Durchmesser von 100 nm bis 1.6 µm hat.

4. Gewebegerüst (1) nach Anspruch 1 oder 2, **dadurch gekennzeichnet, dass** die Kavität (2) einen Durchmesser von 80 nm bis 1.5 µm hat.

5. Gewebegerüst (1) nach einem der vorhergehenden Ansprüche, **dadurch gekennzeichnet, dass** die Nanofaserstruktur (4) Nanofasern mit einem Durchmesser im Bereich von 70 nm bis 121 nm umfasst.

6. Gewebegerüst (1) nach einem der vorhergehenden Ansprüche, **dadurch gekennzeichnet, dass** eine Nano/Mikrokapsel (3) mit mindestens einer biochemischen Markierung, die an der Kavität (2) angebracht ist, sich in mindestens einer der Kavitäten (2) befindet.

7. Gewebegerüst (1) nach einem der vorhergehenden Ansprüche, **dadurch gekennzeichnet, dass** die Nano/Mikrokapsel (3) mit mindestens einem Wachstumsfaktor, der dem Raum (2) zugesetzt ist, sich in mindestens einer der Kavitäten (2) befindet.

8. Verfahren zur Herstellung eines künstlichen biomimetischen neuralen Gewebegerüsts, **dadurch gekennzeichnet, dass** das Verfahren die folgenden Schritte umfasst:
- Herstellen einer Bakterienzelluloselösung in einer bestimmten Konzentration durch Auflösen des Bakterienzellulose-Biopolymers in mindestens einem organischen Lösungsmittel (101),
- Auflösen des synthetischen Polycaprolacton-Polymers in mindestens einem organischen Lösungsmittel zur Herstellung einer Polycaprolactonlösung mit einer bestimmten Konzentration (102),
- Mischen der Bakterienzelluloselösung und der Polycaprolactonlösung in einem vorgegebenen Verhältnis (103),
- Unterziehen des Gemischs, das die Bakterienzelluloselösung und die Polycaprolactonlösung enthält, für eine bestimmte Zeit dem Elektrospinnprozess in mindestens einer Elektrospinnvorrichtung mit mindestens einer Nadel und einer Kollektorplatte durch Einstellen von Variablen wie der Spannung, dem Abstand zwischen der Kollektorplatte und der Nadel und der Durchflussrate, die Auswirkungen auf den Nanofaserdurchmesser und die Morphologie haben (104),
- Sammeln der Polymere, die sich mit dem elektrischen Feld auf der Kollektorplatte in Form von Nanofasern und Nano-/Mikrokapseln während des Elektrospinnens des Gemischs bewegen, um die Nanofasern (4) zu erhalten, die Nano-/Mikrokapseln (3) umfassen (105), und
- Erhalten des Gewebegerüsts (1) durch Trocknen der entstandenen Nanofaserstruktur (4).

9. Verfahren zur Herstellung eines Nervengewebegerüstes (100) nach Anspruch 8, **dadurch gekennzeichnet, dass** eine Bakterienzelluloselösung in einem Lösungsmittel hergestellt wird, so dass die Bakterienzellulose 5 Gew.-% des Lösungsmittels (101) aufweist, und die Polycaprolactonlösung als eine Polycaprolactonpolymerlösung mit 5 Gew.-% in dem Lösungsmittel (102) hergestellt wird.

10. Verfahren zur Herstellung eines Nervengewebegerüstes (100) nach Anspruch 8 oder 9, **dadurch gekennzeichnet, dass** die Bakterienzelluloselösung durch Zugabe des Bakterienzellulose-Biopolymers zu einem Lösungsmittel, das Dimethylformamid (DMF) und Tetrahydrofuran (THF) in einem Gewichtsverhältnis von 50:50 enthält, hergestellt wird.

11. Verfahren zur Herstellung eines Nervengewebegerüstes (100) nach einem der Ansprüche 8 bis 10, **dadurch gekennzeichnet, dass** die Polycaprolactonlösung durch Zugabe des Polycaprolactonpolymers zu dem Lösungsmittelgemisch aus Chloroform und Dimethylformamid (DMF), das in einem Gewichtsverhältnis von 50:50 gemischt wurde, hergestellt wird.

12. Verfahren zur Herstellung eines Nervengewebegerüstes (100) nach einem der Ansprüche 8 bis 11, **dadurch gekennzeichnet, dass** die Bakterienzelluloselösung und die Polycaprolactonlösung im Gewichtsverhältnis 50:50 gemischt werden (103).

13. Verfahren zur Herstellung eines Nervengewebegerüstes (100) nach einem der Ansprüche 8 bis 12, **dadurch gekennzeichnet, dass** das die Bakterienzelluloselösung und die Polycaprolactonlösung enthaltende Gemisch einem Elektrospinnprozess bei einer Durchflussrate von 1 ml/Stunde unterzogen wird (104).

14. Verfahren zur Herstellung eines Nervengewebegerüstes (100) nach einem der Ansprüche 8 bis 13, **dadurch gekennzeichnet, dass** das die Bakterienzelluloselösung und die Polycaprolactonlösung enthaltende Gemisch einem Elektrospinnprozess bei einem Spannungswert von 28 kV unterzogen wird.

15. Verfahren zur Herstellung eines Nervengewebegerüstes (100) nach einem der Ansprüche 8 bis 14, **dadurch gekennzeichnet, dass** das die Bakterienzelluloselösung und die Polycaprolactonlösung enthaltende Gemisch einem Elektrospinnprozess unterzogen wird, so dass der Abstand zwischen der Nadel und der Kollektorplatte 13 cm beträgt.

16. Verfahren zur Herstellung eines Nervengewebegerüstes (100) nach einem der Ansprüche 8 bis 15, **dadurch gekennzeichnet, dass** das die Bakterienzelluloselösung und die Polycaprolactonlösung enthaltende Gemisch einem Elektrospinnprozess für einen Zeitraum von 5 Stunden unterzogen wird.

## Revendications

1. Échafaudage tissulaire neural artificiel tridimensionnel (1) qui est biodégradable et biocompatible, permettant l'autorenouvellement des tissus endommagés, comprenant une structure de nanofibres (4) et comprenant en outre au moins une nano/microcapsule (3) contenant au moins une cavité (2), dans lequel l'échafaudage tissulaire neural artificiel tridimensionnel est obtenu par une méthode comprenant les étapes suivantes :
- préparer une solution de cellulose bactérienne à une concentration donnée en dissolvant le biopolymère de cellulose bactérienne dans au moins un solvant organique (101),
- dissoudre le polymère synthétique polycaprolactone dans au moins un solvant organique pour préparer une solution de polycaprolactone à une certaine concentration (102),
- mélanger la solution de cellulose bactérienne et la solution de polycaprolactone à un rapport prédéterminé (103),
- soumettre le mélange contenant la solution de cellulose bactérienne et la solution de polycaprolactone pendant un certain temps à un processus d'électrofilage dans au moins un dispositif d'électrofilage ayant au moins une aiguille et une plaque collectrice en ajustant des variables telles que la tension, la distance entre la plaque collectrice et l'aiguille et le débit qui a un impact sur le diamètre et la morphologie des nanofibres (104),
- collecter les polymères se déplaçant avec le champ électrique sur la plaque collectrice sous forme de nanofibres et de nano/micro capsules pendant l'électrofilage du mélange de manière à obtenir les nanofibres (4) comprenant des nano/micro capsules (3) (105), et
- obtenir l'échafaudage tissulaire (1) par séchage de la structure de nanofibres résultante (4).

2. Échafaudage tissulaire (1) selon la revendication 1, **caractérisé en ce que** les capsules (3) sont liées les unes aux autres par des fibres nanométriques et macroscopiques.

3. Échafaudage tissulaire (1) selon la revendication 1, **caractérisé en ce que** la nano/microcapsule (3) a un diamètre de 100 nm à 1.6 µm.

4. Échafaudage tissulaire (1) selon la revendication 1 ou 2, **caractérisé en ce que** la cavité (2) a un diamètre de 80 nm à 1.5 µm.

5. Échafaudage tissulaire (1) selon l'une quelconque des revendications précédentes, **caractérisé en ce que** la structure de nanofibres (4) comprend des nanofibres ayant un diamètre compris entre 70 nm à 121 nm.

6. Échafaudage tissulaire (1) selon l'une quelconque des revendications précédentes, **caractérisé en ce qu'**une nano/microcapsule (3) avec au moins une marque biochimique attachée à la cavité (2) située dans au moins une des cavités (2).

7. Échafaudage tissulaire (1) selon l'une quelconque des revendications précédentes, **caractérisé en ce que** la nano/microcapsule (3) avec au moins un facteur de croissance ajouté à l'espace (2) situé dans au moins une des cavités (2).

8. Méthode de production d'un échafaudage tissulaire neural biomimétique artificiel **caractérisée en ce que** ladite méthode comprend les étapes suivantes :
- préparer une solution de cellulose bactérienne à une concentration donnée par dissolution le biopolymère de cellulose bactérienne dans au moins un solvant organique (101),
- dissoudre le polymère synthétique polycaprolactone dans au moins un solvant organique pour préparer une solution de polycaprolactone à une certaine concentration (102)
- mélanger la solution de cellulose bactérienne et la solution de polycaprolactone à un rapport prédéterminé (103),
- soumettre le mélange contenant la solution de cellulose bactérienne et la solution de polycaprolactone pendant un certain temps à un processus d'électrofilage dans au moins un dispositif d'électrofilage ayant au moins une aiguille et une plaque collectrice en ajustant des variables telles que la tension, la distance entre la plaque collectrice et l'aiguille et le débit qui a un impact sur le diamètre et la morphologie des nanofibres (104),
- collecter les polymères se déplaçant avec le champ électrique sur la plaque collectrice sous forme de nanofibres et de nano/micro capsules pendant l'électrofilage du mélange de manière à obtenir les nanofibres (4) comprenant des nano/micro capsules (3) (105), et
- obtenir l'échafaudage tissulaire (1) par séchage de la structure nanofibreuse résultante (4).

9. Méthode de production d'un échafaudage tissulaire nerveux (100) selon la revendication 8, **caractérisée en ce qu'**une solution de cellulose bactérienne est préparée dans un solvant de manière à ce que la cellulose bactérienne représente 5% en poids du solvant (101) et la solution de polycaprolactone est préparée comme une solution de polymère de polycaprolactone de 5% en poids dans le solvant (102).

10. Méthode de production d'un échafaudage tissulaire nerveux (100) selon la revendication 8 ou 9, **caractérisée en ce que** la solution de cellulose bactérienne est préparée en ajoutant le biopolymère de cellulose bactérienne à un solvant contenant du diméthylformamide (DMF) et du tétrahydrofurane (THF) dans un rapport de poids de 50:50.

11. Méthode de production d'un échafaudage tissulaire nerveux (100) selon l'une des revendications 8 à 10, **caractérisée en ce que** la solution de polycaprolactone est préparée en ajoutant le polymère de polycaprolactone au mélange de solvants chloroforme et diméthylformamide (DMF) mélangés dans un rapport de 50:50 en poids.

12. Méthode de production d'un échafaudage tissulaire nerveux (100) selon l'une quelconque des revendications 8 à 11, **caractérisée en ce que** la solution de cellulose bactérienne et la solution de polycaprolactone sont mélangées à raison de 50:50 en poids (103).

13. Méthode de production d'un échafaudage tissulaire nerveux (100) selon l'une des revendications 8 à 12, **caractérisée en ce que** le mélange contenant la solution de cellulose bactérienne et la solution de polycaprolactone est soumis à un processus d'électrofilage à un débit de 1 ml/hr (104).

14. Méthode de production d'un échafaudage tissulaire nerveux (100) selon l'une quelconque des revendications 8 à 13, **caractérisée en ce que** le mélange comprenant la solution de cellulose bactérienne et la solution de polycaprolactone est soumis au processus d'électrofilage à une valeur de tension de 28 kV.

15. Méthode de production d'un échafaudage tissulaire nerveux (100) selon l'une quelconque des revendications 8 à 14, **caractérisée en ce que** le mélange comprenant la solution de cellulose bactérienne et la solution de polycaprolactone est soumis à un processus d'électrofilage de manière à ce que la distance entre l'aiguille et la plaque collectrice est 13 cm.

16. Méthode de production d'un échafaudage tissulaire nerveux (100) selon l'une des revendications 8 à 15, **caractérisée en ce que** le mélange contenant la solution de cellulose bactérienne et la solution de polycaprolactone est soumis à un processus d'électrofilage pendant une période de 5 heures.
